Europäisches Patentamt

European Patent Office

Office européen des brevets

(11) Veröffentlichungsnummer: **0 295 532**
**A2**

(12) **EUROPÄISCHE PATENTANMELDUNG**

(21) Anmeldenummer: 88109031.0

(22) Anmeldetag: 07.06.88

(51) Int. Cl.⁴: **G09B 21/00** , **A61F 9/08**

(30) Priorität: 19.06.87 DE 3720276

(43) Veröffentlichungstag der Anmeldung:
**21.12.88 Patentblatt 88/51**

(84) Benannte Vertragsstaaten:
**AT BE CH DE FR GB IT LI LU NL SE**

(71) Anmelder: **Brümmer, Hans, Prof. Dr.-Ing.**
**Steinberg 12**
**D-3257 Springe 1(DE)**

(72) Erfinder: **Brümmer, Hans, Prof. Dr.-Ing.**
**Steinberg 12**
**D-3257 Springe 1(DE)**

(74) Vertreter: **Thömen, Uwe, Dipl.-Ing.**
**Patentanwalt U. Thömen Zeppelinstrasse 5**
**D-3000 Hannover 1(DE)**

(54) **Vorrichtung zur Ausgabe von Schriftzeichen und Symbolen durch elektrische Reizstromimpulse.**

(57) Vorrichtung zur Ausgabe von Schriftzeichen und Symbolen durch elektrische Reizstromimpulse mit Reizelektroden, die Punktelektroden und eine gemeinsame Gegenelektrode umfassen, wobei bei gleichzeitiger Berührung von Punktelektrode und Gegenelektrode durch die menschliche Haut elektrische Reizströme fließen. Die Reizelektroden werden mit rechteckförmigen Wechselströmen mit einstellbarer konstanter Amplitude angesteuert. Jeder Reizstromimpuls wird zur Vermeidung einer zu starken Reizung dann abgeschaltet, wenn die Anstiegsgeschwindigkeit der Spannung an den Reizelektroden einen vorbestimmten Wert überschreitet. Alternativ kann jeder Reizstromimpuls zur Vermeidung einer zu starken Reizung dann abgeschaltet werden, wenn der Betrag des Reizstromes an der Punktelektrode ungleich dem Betrag des Stromes an der zugehörigen Gegenelektrode ist.

Fig. 1

## Vorrichtung zur Ausgabe von Schriftzeichen und Symbolen durch elektrische Reizstromimpulse

Die Erfindung betrifft eine Vorrichtung zur Ausgabe von Schriftzeichen und Symbolen durch elektrische Reizstromimpulse gemäß dem Oberbegriff der Patentansprüche 1 und 2.

Solche Vorrichtungen sollen blinden oder stark sehbehinderten Personen Text- und Bildinformationen, die beispielsweise von datenverarbeitenden Geräten üblicherweise auf Bildschirmen ausgegeben werden, über Elektrodenanordnungen als tastbare Information zur Verfügung stellen. Die Übermittlung der Information erfolgt bei Berührung von Reizelektroden durch elektrische Reizstromimpulse, wobei dafür zu sorgen ist, daß die dabei auftretende Empfindung der Person einen vorgebbaren Wert nicht übersteigt.

Es ist bekannt, daß für die Ausgabe von Texten Anordnungen von elektromagnetisch oder elektrostriktiv (Piezo-Effekt) angehobenen Taststiften verwendet werden. Dabei kann die Ausgabe sowohl in Form bekannter Schriftzeichen als auch in der Blindenschrift erfolgen.

Diese Anordnungen sind technisch sehr aufwendig, im Betrieb nicht sehr zuverlässig und außerdem teuer in der Herstellung.

Ferner sind Anordnungen von Reizelektroden bekannt, mit deren Hilfe grob gerasterte Fernsehbilder (z.B. 32 x 32 Bildpunkte) auf die menschliche Bauchhaut übertragen werden können ("Functional Electrical Stimulation" (Edited by Hambrecht & Reswick), Biomedical Engineering and Instrumentation, Volume 3, 1975. Dort: Seite 289...301: Collins, C.C.: "Electrotactile visual prosthesis" sowie Seite 303...309: Saunders, F.A.: "Recommended precedures for electrocutaneous displays"; und US-PS 3,848,608).

Die in dieser Anordnung verwendeten elektronischen Ansteuerschaltungen sind nur geeignet für elektrisch homogene Hautbereiche, wie z.B. der Bauchhaut. Für die Ausgabe von Schriftzeichen durch Berührung von Reizelektroden mit den Fingerspitzen lassen sich die bekannten Anordnungen demgegenüber nicht verwenden, weil die Haut im Bereich der Fingerspitzen nicht homogen ist. Außerdem mangelt es den bekannten Anordnungen an Schutzmaßnahmen gegen unangenehme Reize, die beispielsweise bei einem geringen Kontaktdruck zwischen der Haut und den Reizelektroden auftreten können, oder die bei Fehlerströmen auftreten, wenn diese über die Nerven oder Blutbahnen zu benachbarten Elektrodenanordnungen fließen.

Der Erfindung liegt die Aufgabe zugrunde, eine Vorrichtung mit elektrischer Reizung der menschlichen Haut zu schaffen, die eine Ausgabe von Schriftzeichen und Symbolen durch elektrische Reizstromimpulse ermöglicht, die mit den Fingerspitzen auf Elektrodenanordnungen wahrgenommen werden können, wobei zu starke unangenehme Reize vermieden sind.

Die Lösung dieser Aufgabe erfolgt durch die Merkmale der Patentansprüche 1 oder 2.

Bei der Erfindung werden also Konstantstromquellen zur Erzeugung steilflankiger Reizstromimpulse mit Rechteckform verwendet, die sich als vorteilhaft für die elektrisch sehr unterschiedlich empfindlichen Bereiche der vorderen Fingerglieder bzw. der Haut an den Fingerspitzen erwiesen haben. Außerdem sind Schaltungsmaßnahmen gegen unangenehme Reize vorgesehen, die z.B. bei geringem Kontaktdruck zwischen der Haut und der Elektrodenanordnung, bei gleitender Kontaktberührung oder bei Fehlerströmen auftreten, wenn diese über die Nerven oder Blutbahnen zu benachbarten Elektrodenanordnungen fließen.

Die beim Abtasten eventuell auftretenden unangenehmen elektrischen Reizstärken werden also durch spezielle Schaltungsmaßnahmen weitgehend vermieden. Dies wird dadurch ermöglicht, daß jeder Reizstromimpuls dann abgeschaltet ist, wenn die Anstiegsgeschwindigkeit der Spannung an den Reizelektroden einen vorbestimmten Wert überschreitet. Eine andere Lösung besteht darin, den Reizstrom dann abzuschalten, wenn der Betrag des Reizstromes an der Punktelektrode ungleich dem Betrag des Stromes an der zugehörigen Gegenelektrode ist.

Die Elektrodenanordnungen der erfindungsgemäßen Vor richtung lassen sich vorteilhaft in Form gedruckter Schaltungen realisieren.

Die mit der Erfindung erzielbaren Vorteile bestehen insbesondere auch darin, daß die bekannten teuren, magnetisch oder elektrostriktiv angehobenen Tastelemente durch Reizelektroden ersetzt werden, welche durch billige und zuverlässige gedruckte Schaltungen herstellbar sind. Die Reizung erfolgt durch die steilflankigen Reizwechselströme mit sehr geringen elektrischen Ladungsmengen. Die gewünschte Reizintensität ist einstellbar. Durch die erwähnten Schutzmaßnahmen wird dafür gesorgt, daß bei Veränderung des Kontaktdruckes, bei gleitender Kontaktgabe sowie bei Fehlerströmen über andere Körperbereiche keine unangenehmen Reizstärken auftreten können.

Ein bevorzugtes Ausführungsbeispiel der Erfindung ist in den Zeichnungen dargestellt und wird im folgenden näher beschrieben. Es zeigen:

Fig. 1 Das Prinzip der Ansteuerung einer Reizelektrode und ein Ersatzschaltbild der menschlichen Haut,

Fig. 2 eine Gruppe von Reizstromimpulsen und den Verlauf der Elektrodenspannung bei symmetrischer Spannungsversorgung,

Fig. 3 eine Gruppe von Reizstromimpulsen und den Verlauf der Elektrodenspannung bei unsymmetrischer Spannungsversorgung,

Fig. 4 das Prinzip einer Ansteuerschaltung für zwei Elektrodenanordnungen,

Fig. 5 ein Impulsdiagramm zu Fig. 4,

Fig. 6 eine erste Abwandlung der Ansteuerschaltung gemäß Fig. 4,

Fig. 7 eine zweite Abwandlung der Ansteuerschaltung gemäß Fig. 4,

Fig. 8 den Verlauf der Elektrodenspannung bei unterschiedlichem Kontaktdruck bzw. unterschiedlicher Kontaktfläche,

Fig. 9 das Prinzip einer Schnellabschaltung für hohe Anstiegsgeschwindigkeit der Reizelektrodenspannung sowie für Fehlerströme,

Fig. 10 eine Elektrodenanordnung mit dreiseitiger Umschließung der Punktelektrode und unterschiedlichem Elektrodenabständen, und

Fig. 11 eine Elektrodenanordnung gemäß einer anderen Ausführungsform.

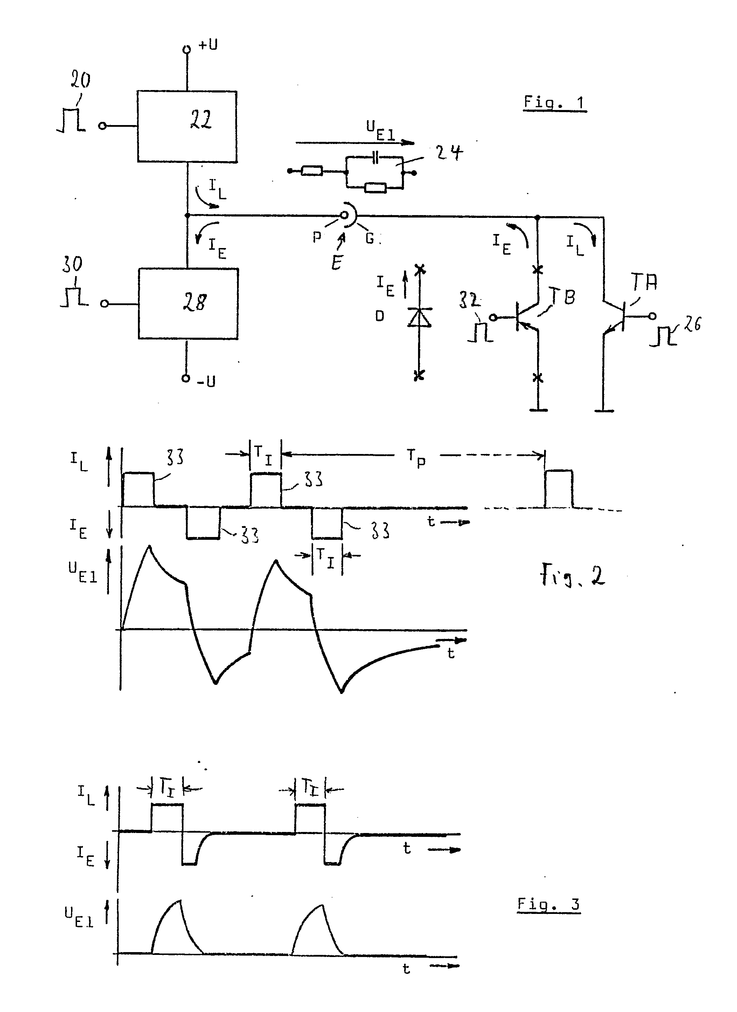

In Fig. 1 ist das Prinzip der Ansteuerung von Reizelektroden dargestellt, wobei die entsprechende Elektrodenanordnung E eine Punktelektrode P und eine Gegenelektrode G umfaßt. Die Punktelektrode P ist mit einer Ladestromquelle 22 verbunden, welche mit einem Schaltsignal 20 zum Ein- und Ausschalten angesteuert ist. Wenn die Reizelektroden P, G mit den Fingerspitzen durch die menschliche Haut überbrückt werden, kann zunächst ein Ladestrom $I_L$ über die menschliche Haut und über den Transistor TA fließen, der über einen Anschluß mit einem anderen Schaltsignal 26 angesteuert ist.

Oberhalb der Elektrodenanordnung E ist in Fig. 1 das elektrische Ersatzschaltbild 24 der menschlichen Haut dargestellt. An der Punktelektrode P und der Gegenelektrode G liegt die Spannung $U_{E1}$ an.

Die Punktelektrode P ist außerdem mit einer Entladestromquelle 28 verbunden, die über eine Eingangsklemme mit einem Schaltsignal 30 zum Ein- und Ausschalten angesteuert ist. Wenn der Reizstrom $I_L$ geflossen ist, werden die Ladestromquelle 22 und der Transistor TA abgeschaltet, und es wird die Entladestromquelle 28 mit dem Schaltsignal 30 eingeschaltet. Außerdem wird jetzt ein anderer Transistor TB über ein Schaltsignal 32 eingeschaltet, so daß der Strom $I_E$ fließen kann, wenn die Elektrodenanordnung E durch die menschliche Haut überbrückt wird. Für die Ladestromquelle 22 und die Entladestromquelle 28 können bekannte elektronische Schaltungen verwendet werden.

Fig. 2 zeigt den zeitlichen Verlauf der Ströme

$I_L$ und $I_E$ und der Spannung $U_{E1}$ an den Reizelektroden P, G. Die Impulszeit $T_I$ sollte nicht größer sein als die Chronaxie der menschlichen Nerven (0,1...1 ms). Wählt man die Impulspausenzeit $T_P$ zwischen zwei Impulsgruppen kleiner als 20 ms, wirkt der an den Reizelektroden P, G empfundene Reiz "klopfend", während ein "kribbelnder" Reiz entsteht, wenn die Impulspausenzeit $T_P$ größer oder gleich 20 ms ist. Eine Impulsgruppe kann aus einem oder mehreren positiven und negativen Impulswechseln bestehen. Die Impulspausenzeit $T_P$ kann auch zwischen einem positiven und einem negativen Einzelimpuls (Reizstromimpuls 33) auftreten.

In Fig. 1 ist die Ladestromquelle 22 mit einer positiven Betriebsspannung +U und die Entladestromquelle 28 mit einer negativen Betriebsspannung -U verbunden. Wird die negative Betriebsspannung -U der Entladestromquelle 28 durch das Bezugspotential 0 V ersetzt, ändern sich die Ströme $I_L$, $I_E$ und die Spannung $U_{E1}$ wie in Fig. 3 dargestellt. Dabei kann in Fig. 1 der Transistor TB durch eine Diode D ersetzt werden.

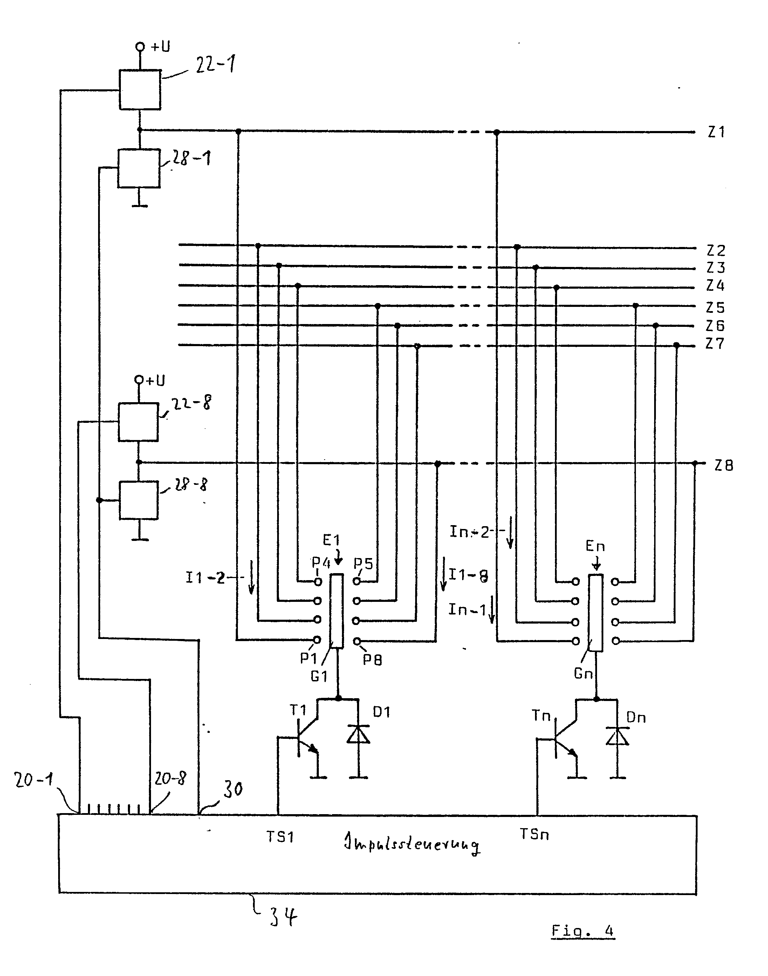

Fig. 4 zeigt ein Ansteuerungsprinzip mit n Elektrodenanordnungen E1 - En, von denen jede beispielsweise aus acht Punktelektroden P1 - P8 und einer Gegenelektrode G1 - Gn besteht. Jede Punktelektrode P1 - P8 ist mit einer entsprechenden Zeilenleitung Z1 - Z8 verbunden. Für die Zeilenleitungen ist je eine Ladestromquelle 22-1 bis 22-8 und eine Entladestromquelle 28-1 bis 28-8 vorhanden. Die Auswahl einer bestimmten Elektrodenanordnung E1 - En erfolgt durch eine Impulssteuerung 34.

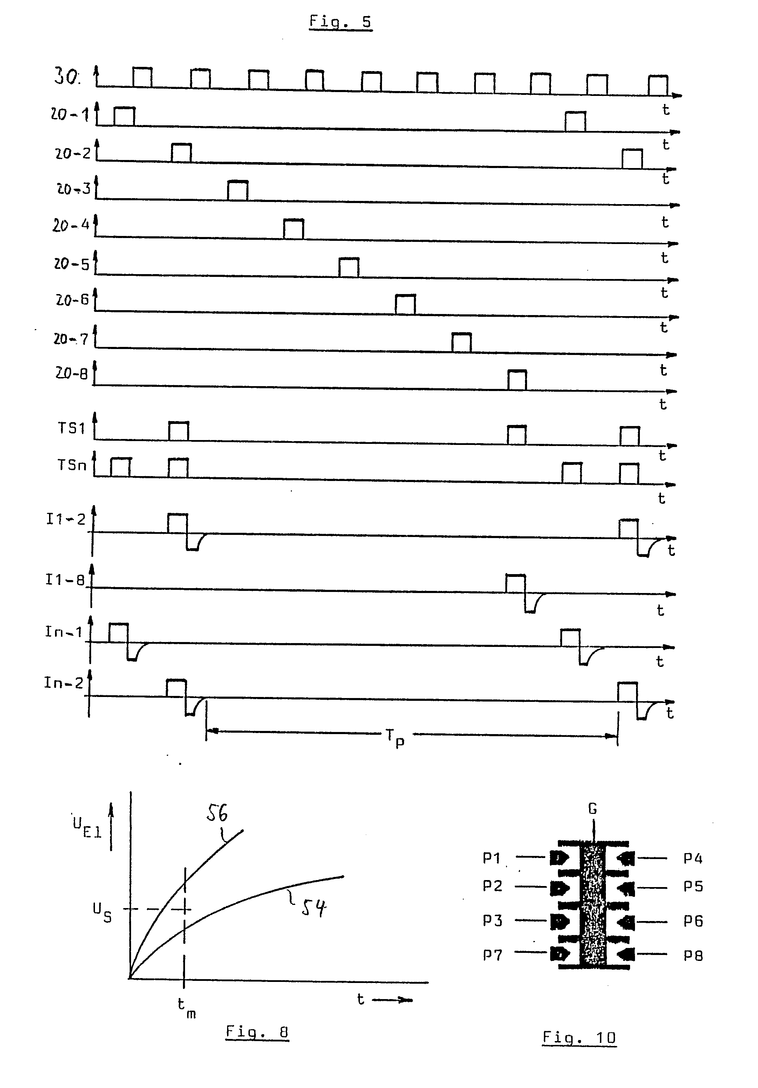

In Fig. 5 ist das Ansteuerungsprinzip durch die Impulssteuerung 34 in einem Zeitdiagramm dargestellt, wobei zur Vereinfachung nur ein Impulswechsel je Impulsgruppe verwendet wird. Die Bezeichnungen in Fig. 5 beziehen sich auf Fig. 4.

Die Ladestromquellen 22-1 bis 22-8 werden zeitlich nacheinander durch die Schaltsignale 20-1 bis 20-8 angesteuert. Ein Reizstrom kann nur fließen, wenn die Gegenelektroden G1 - Gn einer Elektrodenanordnung E1 - En über Schaltsignale TS1 - TSn der Impulssteuerung 34 angesteuert und gleichzeitig eine Verbindung von einer der Punktelektroden P1 - P8 und einer der angesteuerten Gegenelektroden G1 - Gn über die menschliche Haut vorhanden ist. Die Schaltsignale TS1 - TSn werden zugeordneten Schaltungen zugeführt, die jeweils durch Transistoren T1 - Tn mit parallel geschalteten Dioden D1 - Dn bestehen.

Im dargestellten Beispiel kann in Fig. 4 ein Reizstrom an den Punktelektroden P2 und P8 (Ströme I1-2 und I1-8) der Elektrodenanordnung E1 sowie an den Punktelektroden P1 und P2 (Ströme In-1 und In-2) der Elektrodenanordnung En entstehen. Die Entladestromquellen 28-1 bis 28-8 werden nach jeder Ansteuerung einer Ladestromquelle 22-1 bis 22-8 mit dem Schaltsignal 30 angesteuert.

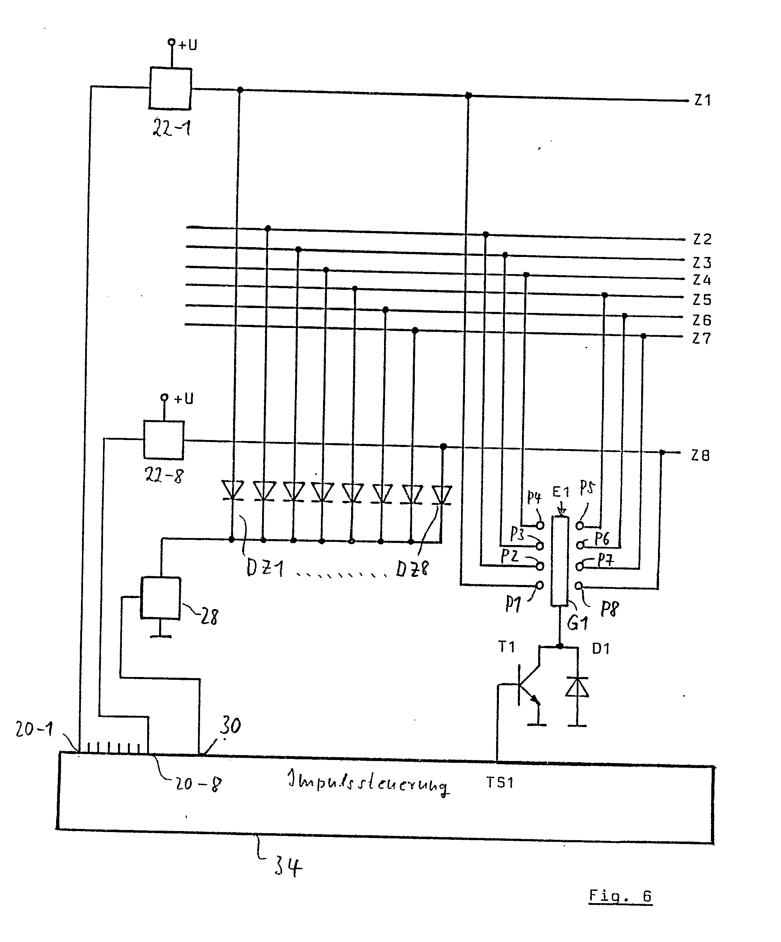

Der Schaltungsaufwand läßt sich auf eine einzige Entladestromquelle 28 verringern, wenn diese gemäß Fig. 6 über die Dioden DZ1 - DZ8 an die entsprechenden Zeilenleitungen Z1 - Z8 angekoppelt wird.

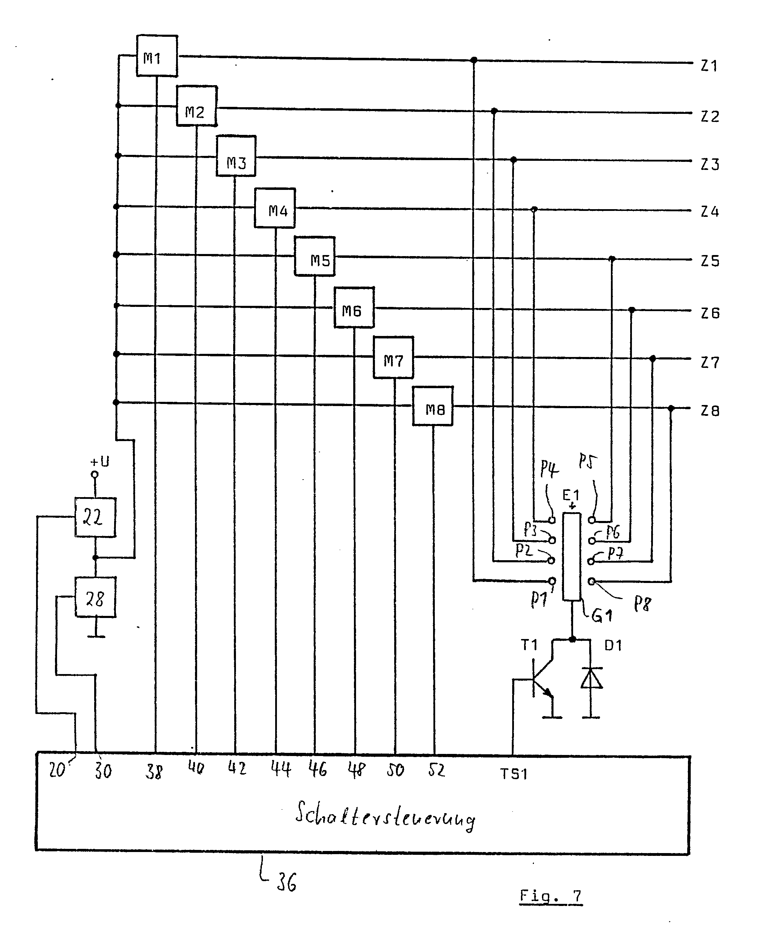

Eine weitere Möglichkeit zur Reduzierung des Schaltungsaufwandes zeigt Fig. 7. Dort wird je eine Ladestromquelle 22 bzw. Entladestromquelle 28 zeitlich nacheinander über bidirektionale Multiplexschalter M1 - M8 auf die Zeilenleitungen Z1 - Z8 geschaltet. Die Aktivierung der Multiplexschalter M1 - M8 erfolgt durch Schaltsignale 38 - 52 einer Schaltersteuerung 36.

Die erfindungsgemäßen Schaltungsprinzipien sind ergänzt durch Schaltungsmaßnahmen gegen zu starke Reizintensitäten. Die Reizstärke wird bei einem bestimmten Kontaktdruck zwischen dem tastenden Finger und den Elektrodenanordnungen eingestellt, beispielsweise mit Hilfe von Potentiometern, wobei zweckmäßigerweise allen Punktelektroden, die in einer Reihe nebeneinander liegen, ein Potentiometer zuzuordnen ist.

Die nach kurzer Zeit auftretende Adaption der Nerven an die Reizstärke kann über ein zusätzliches Potentiometer, das auf alle Punktelektroden gleichzeitig wirkt, ausgeglichen werden. Die Reizintensität ergibt sich aus der gesamten elektrischen Ladung einer Impulsgruppe (vgl. Fig. 2). Zur Einstellung der Reizstärke eignet sich besonders die Impulsdauer $T_I$ der Reizstromimpulse in Fig. 2.

Beim Nachlassen der Kontaktkraft bzw. bei gleitender Bewegung treten erhöhte Stromdichten in der Haut auf, was zu größeren Reizintensitäten führt. Dies kann starke Änderungen der elektrischen Werte des Ersatzschaltbildes 24 der Haut (vgl. Fig. 1) ergeben, woraus eine Vergrößerung der Anstiegsgeschwindigkeit der Spannung $U_{E1}$ über den Reizelektroden P, G resultiert.

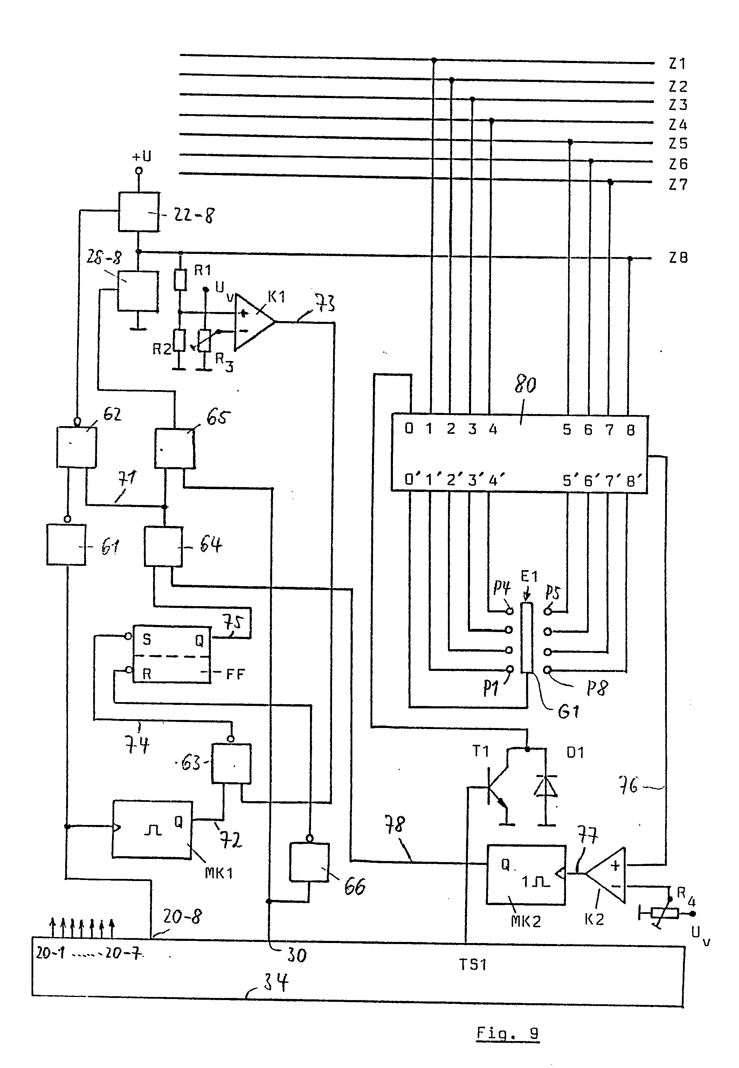

In Fig. 8 zeigt die Kurve 54 den Anstieg der Elektrodenspannung UE1 bei normalem Kontaktdruck, während die Kurve 56 den Spannungsverlauf bei reduziertem Kontaktdruck bzw. bei einer Verringerung der Kontaktfläche darstellt (vgl. auch Fig. 3, wo außer dem Anstieg auch der komplette Spannungsverlauf von $U_{E1}$ dargestellt ist).

Das Prinzip der im folgenden beschriebenen Schaltung gemäß Fig. 9 beruht darauf, daß die Spannung $U_{E1}$ zu einer Zeit $t_m$ (vgl. Fig. 8) gemessen wird, bei der die Reizstromimpulse 33 bei optimaler Berührung zwischen Haut und Elektrodenanordnung noch keine spürbare Reizempfindung auslösen. Unter dieser Bedingung bleibt die Elektrodenspannung bei $t = t_m$ unter dem Wert einer Schwellenspannung $U_S$. Ist bei der Messung zur Zeit $t_m$ die Elektrodenspannung $U_{E1}$ größer als $U_S$, wird der Reizstromimpuls sofort abgeschaltet.

Diese Messung erfolgt bei jedem positiven Reizstromimpuls.

Eine Schaltung zur Schnellabschaltung bei zu hohen Reizstärken ist für jede Kombination von Ladestromquelle und Entladestromquelle erforderlich. In Fig. 9 wird das Prinzip am Beispiel der Ansteuerung der Zeile Z8 erläutert. Das Schaltbild entspricht prinzipiell Fig. 4. Die Ladestromquelle 22-8 wird durch das Schaltsignal 20-8 über einen Inverter 61 und ein NOR-Gatter 62 eingeschaltet. Eine Leitung 71 liegt zunächst auf dem Pegel Low (L). Gleichzeitig wird der Ausgang 72 der monostabilen Kippstufe MK1 für die Zeit $t_m$ (Fig. 8) auf den Pegel High (H) gesetzt.

Die Elektrodenspannung, die identisch mit der Spannung auf der Zeilenleitung Z8 ist, wird über einen Spannungsteiler R1, R2 auf den positiven Eingang des Komparators K1 geschaltet und mit der über den Widerstand R3 reduzierten Vergleichsspannung $U_V$ verglichen. Die Spannung am negativen Eingang des Komparators K1 entspricht der um das Teilerverhältnis des Spannungsteilers R1, R2 reduzierten Schwellenspannung $U_S$ aus Fig. 8. Übersteigt die Spannung $U_{E8}$ auf der Zeilenleitung Z8 den Wert der Schwellenspannung $U_S$, - schaltet der Ausgang 73 des Komparators K1 von dem Pegel L auf den Pegel H. Erfolgt dieses Umschalten bei $t < t_m$, wird der Ausgang 74 des NAND-Gatters 63 auf den Pegel L gesetzt und am Ausgang 75 des Flip-Flops FF erscheint ein H-Pegel. Über das ODER-Gatter 64 und das NOR-Gatter 62 wird die Ladestromquelle 22-8 abgeschaltet, und über das ODER-Gatter 65 wird die Entladestromquelle 28-8 eingeschaltet.

Der zyklisch wiederkehrende Schaltimpuls 30 setzt den Ausgang des Flip-Flops FF über den Inverter 66 zurück.

Die Erfindung umfaßt eine weitere Schutzmaßnahme gegen Körperströme, die bei Berührung mit beiden Händen auftreten können. Wird beispielsweise mit einem Finger die Punktelektrode P2 der Elektrodenanordnung E1 (Fig. 4) und mit einem anderen Finger der selben oder der anderen Hand die Gegenelektrode Gn der Elektrodenanordnung En berührt, so kann bei Ansteuerung der Zeile Z2 und des Transistors Tn ein Körperstrom fließen. Dieser fließt bevorzugt in Longlitudinalrichtung in den niederohmigen Blut- und Nervenbahnen des menschlichen Körpers und erzeugt unangenehme Reizstärken. Diese Körperströme sind bei der Erfindung aufgrund der Strombegrenzung und sehr kurzen Impulsdauern ungefährlich.

Bei der erfindungsgemäßen Schutzmaßnahme wird in jeder Elektrodenanordnung der zufließende Strom mit dem abfließenden Strom in einem an sich bekannten Vergleicher 80 (Fig. 9) verglichen. Der Vergleich kann beispielsweise erfolgen, indem die Verbindungsleitungen zwischen den Zeilenleitungen Z1 - Z8 und den zugehörigen Punktelektroden P1 - P8 sowie der Anschluß der Gegenelektrode G1 durch verschiedene Primärwicklungen eines im Vergleicher 80 enthaltenen Übertragers geleitet werden. Dabei fließt der Strom der Gegenelektrode G1 gegensinnig zu den Strömen der Punktelektroden P1 - P8. Wenn die Ströme Unterschiede aufweisen, wird in einer Sekundärwicklung des erwähnten Übertragers eine Spannung induziert. Diese wird über die Leitung 76 dem Komparator K2 zugeführt. Ist diese Spannung größer als die an dem Widerstand R4 eingestellte Vergleichsspannung, schaltet der Ausgang 77 auf H-Potential und löst damit eine monostabile Kippstufe MK2 aus. Deren Ausgangssignal H sperrt über eine Leitung 78 die Ladestromquelle 22-8 und gibt die Entladestromquelle 28-8 über das ODER-Gatter 65 frei. Über die Zeitdauer des H-Ausgangssignals der monostabilen Kippstufe MK2 läßt sich die Erzeugung weiterer Reizstromimpulse für eine definierte Zeit sperren.

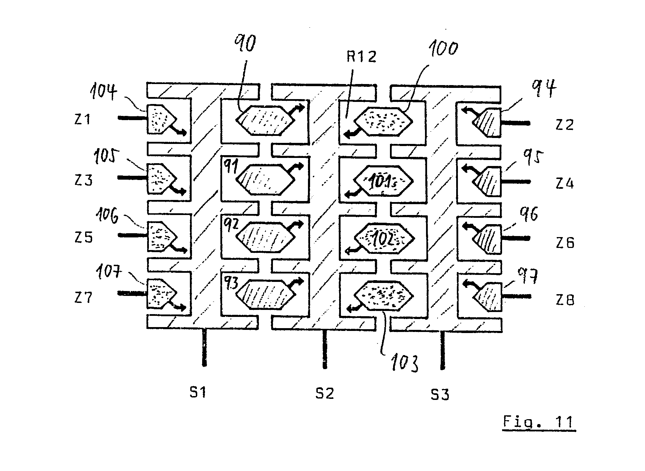

Die Eindringtiefe der Reizströme in die Haut hängt von der Dicke der äußeren Hautschichten (Epidermis und Corium), ihrem Widerstand, dem Elektrodenabstand der Reizelektroden und der Stärke der Reizströme ab. Schichtdicke und Widerstand nebeneinanderliegender Hautbereiche können im Bereich der Fingerspitzen sehr unterschiedlich sein. Die Erfindung umfaßt eine Elektrodenform, die aufgrund der unterschiedlichen Elektrodenabstände zwischen der punktförmigen Elektrode P und der größerflächigen Gegenelektrode G örtlich unterschiedliche Eindringtiefen des Reizstromes erzeugt. Die Reizempfindung entsteht dabei zwischen den Spitzen der Elektroden P1 - P8 und dem inneren Teil der Gegenelektrode G. Fig. 10 zeigt eine für die Darstellung eines Blindenschriftzeichens mit acht Punkten geeignete Anordnung.

Zusammenhängende zweidimensionale Elektrodenanordnungen werden entsprechend Fig. 11 gestaltet, welche einen Aufbau zeigt, bei dem mehrere der Anordnungen nach Fig. 10 nebeneinander plaziert sind. Die Zuordnung der innenliegenden - schraffierten Elektroden 90 -97 und der gepunkteten Elektroden 100 - 107 zu den Zeilenleitungen Z1 - Z8 ist durch Pfeile angedeutet. Die Pfeilspitzen deuten jeweils eine elektrische Verbindung an. So ist beispielsweise die Elektrode 104 mit der Elektrode 100, die Elektrode 105 mit der Elektrode 101 usw. verbunden. Entsprechend ist die Elektrode 94 mit der Elektrode 90, die Elektrode 95 mit der Elektrode 91 usw. verbunden. Eine Kombination mit einer Schaltung zur Erkennung von Fehlerströmen (entsprechend dem Vergleicher 80 in Fig. 9) mit nachgeschalteter Schnellabschaltung ist möglich.

Wird in Fig. 11 beispielsweise der Zeilenanschluß Z1 mit einer Ladestromquelle bzw. Entladestromquelle verbunden und von den Spaltenanschlüssen S1 - S3 der Spaltenanschluß S2 über Halbleiterschalter auf Massepotential gelegt, so entsteht der elektrische Reiz im aufliegenden Finger über der Stelle bzw. dem Bereich R12.

**Ansprüche**

1. Vorrichtung zur Ausgabe von Schriftzeichen und Symbolen durch elektrische Reizstromimpulse mit einer Mehrzahl von Elektrodenanordnungen mit einer Ansteuerschaltung, wobei die Elektrodenanordnungen Reizelektroden umfassen, die jeweils aus Punktelektroden und größerflächigen Gegenelektroden bestehen, und wobei bei gleichzeitiger Berührung von Punktelektroden und Gegenelektrode durch die menschliche Haut elektrische Reizströme fließen, dadurch gekennzeichnet, daß die Ansteuerschaltung (22, 28, 34) so ausgebildet ist, daß die Reizströme ($I_L$, $I_E$) als steilflankige Wechselströme mit einstellbarer konstanter Amplitude fließen, wenn die jeweilige Punktelektrode (P) und die zugehörige Gegenelektrode (G) angesteuert sind, wobei jeder Reizstromimpuls (33) zur Vermeidung zu starker Reizung dann abgeschaltet ist, wenn die Anstiegsgeschwindigkeit der Spannung ($U_{E1}$) an den Reizelektroden (P; G) einen vorbestimmten Wert überschreitet.

2. Vorrichtung zur Ausgabe von Schriftzeichen und Symbolen durch elektrische Reizstromimpulse mit einer Mehrzahl von Elektrodenanordnungen mit einer Ansteuerschaltung, wobei die Elektrodenanordnungen Reizelektroden umfassen, die jeweils aus Punktelektroden und größerflächigen Gegenelektroden bestehen, und wobei bei gleichzeitiger Berührung von Punktelektroden und Gegenelektrode durch die menschliche Haut elektrische Reizströme fließen, dadurch gekennzeichnet, daß die Ansteuerschaltung (22, 28, 34) so ausgebildet ist, daß die Reizströme ($I_L$, $I_E$) als steilflankige Wechselströme mit einstellbarer konstanter Amplitude fließen, wenn die jeweilige Punktelektrode (P) und die zugehörige Gegenelektrode (G) angesteuert sind, wobei jeder Reizstromimpuls (33) zur Vermeidung zu starker Reizung dann abgeschaltet ist, wenn der Betrag des Reizstromes ($I_L$, $I_E$) an der Punktelektrode (P) ungleich dem Betrag des Stromes an der zugehörigen Gegenelektrode (G) ist.

3. Vorrichtung nach Anspruch 1 und/oder 2, dadurch gekennzeichnet, daß die Punktelektroden (P1 -P8) der Elektrodenanordnung zeitlich nacheinander angesteuert und dabei zuerst mit einer Ladestromquelle (22) und danach zur Vermeidung einer Gleichstromkomponente mit einer Entladestromquelle (28) verbunden sind, wobei während dieser Ansteuerung die Strompfade bei Berührung über die menschliche Haut sowie über mit den Gegenelektroden (G) verbundene Halbleiterschalter (TA, TB) geschlossen werden.

4. Vorrichtung nach Anspruch 1 und/oder 2, dadurch gekennzeichnet, daß die Ladestromquelle (22) und die Entladestromquelle (28) mit der Punktelektrode (P) und die Halbleiterschalter (TA, TB) mit den Gegenelektroden (G) verbunden sind.

5. Vorrichtung nach einem der vorhergehenden Ansprüche 1 - 4, dadurch gekennzeichnet, daß die Punktelektroden (P1 - P8) eine Anordnung bilden, die den Punkten der Blindenschrift entspricht.

6. Vorrichtung nach einem der vorhergehenden Ansprüche 1 - 4, dadurch gekennzeichnet, daß durch die Anordnung der Punktelektroden (P1 - P8) Buchstaben, Ziffern und Satzzeichen aller üblichen Schriftarten sowie Symbole und bildhafte Darstellungen darstellbar sind.

7. Vorrichtung nach einem der vorhergehenden Ansprüche 1 - 6, dadurch gekennzeichnet, daß einzelne Punktelektroden (P1 - P8) einer Elektrodenanordnung oder eine Gruppe von Punktelektroden einer Elektrodenanordnung besonders hervorgehoben sind, indem diese mit Impulsen (33) oder Impulsgruppen angesteuert sind, deren zeitlicher Abstand ($T_P$) voneinander so groß gewählt ist, daß der Benutzer einen Klopfreiz wahrnimmt, während der zeitliche Abstand ($T_P$) der Impulse oder Impulsgruppen, mit denen die restlichen Elektrodenpunkte bzw. Elektrodenanordnungen angesteuert sind, geringer ist.

8. Vorrichtung nach einem der vorhergehenden Ansprüche 1 - 7, dadurch gekennzeichnet, daß die Gegenelektrode (G) die Punktelektroden (P1 - P8) auf drei Seiten oder allseitig umschließt, wobei die Abstände zwischen der Gegenelektrode (G) und den Punkt elektroden (P1 - P8) zur Erzielung unterschiedlicher Eindringtiefen des Reizstromes in die Haut verschieden sind.

9. Vorrichtung nach Anspruch 1 und/oder 2, dadurch gekennzeichnet, daß bei vorgegebener konstanter Amplitude der Reizströme ($I_E$, $I_L$) die Impulsdauer ($T_I$) auf wählbare Werte einstellbar ist, wodurch die Ladungsmenge (Produkt aus Strom und Zeit) der Reizstromimpulse vorgegeben wird.

Fig. 1

Fig. 2

Fig. 3

Fig. 4

## Fig. 5

## Fig. 8

## Fig. 10

+U

22-1

Z1
Z2
Z3
Z4
Z5
Z6
Z7

+U

22-8

Z8

P4 E1 P5
P3 P6
P2 P7
P1 P8
G1

DZ1 .......... DZ8

28

T1 D1

20-1

30

20-8    Impulssteuerung    TS1

34

<u>Fig. 6</u>

Fig. 7

Fig. 9

Fig. 11